# EUROPÄISCHE PATENTSCHRIFT

(11) **EP 2 785 467 B1**
(45) Veröffentlichungstag und Bekanntmachung des Hinweises auf die Patenterteilung: **30.12.2015**
(21) Anmeldenummer: 12818469.4
(22) Anmeldetag: 30.11.2012
(51) Int. Cl.: A61L 9/14, B05B 7/00, B05B 17/06

(54) **ZERSTÄUBUNGSVORRICHTUNG**
ATOMISATION DEVICE
DISPOSITIF D'ATOMISATION

(30) Priorität: 02.12.2011 AT 17892011
(43) Veröffentlichungstag der Anmeldung: 08.10.2014
(73) Patentinhaber: Braincon Handels GmbH, 1190 Wien (AT)
(72) Erfinder: LJUHAR, Davul, A-1190 Wien (AT)
(74) Vertreter: Ellmeyer, Wolfgang
(86) Internationale Anmeldenummer: PCT/AT2012/050188
(87) Internationale Veröffentlichungsnummer: WO 2013/078495

(56) Entgegenhaltungen:
- WO-A1-2009/013843
- DE-B3-102005 019 686
- GB-A- 525 736

## Beschreibung

Die Erfindung betrifft eine Zerstäubungsvorrichtung mit einer Zerstäubungskammer zur Aufnahme einer Flüssigkeit und zumindest einem Vernebler zum Zerstäuben der Flüssigkeit zu Flüssigkeitströpfchen sowie einer Austrittsöffnung für den Austritt des so erzeugten Flüssigkeitsdampfes oder -nebels aus der Zerstäubungskammer, wobei der aus der Flüssigkeit aufsteigende Flüssigkeitsdampf oder - nebel über eine Druckvorrichtung in Richtung zur Austrittsöffnung bewegt wird, und wobei zumindest eine Vorrichtung zur Umlenkung des Flüssigkeitsdampfes oder - nebels vorgesehen ist, welche im Bereich oberhalb der Flüssigkeitsoberfläche angeordnet ist, wobei im Bereich der Austrittsöffnung ein Beschleunigungsprisma ausgebildet ist, das über den Flüssigkeitspegel hinausragt und so angeordnet ist, dass eine Querschnittsverengung der Zerstäubungskammer ausgebildet ist, und wobei die zumindest eine Vorrichtung zur Umlenkung des Flüssigkeitsdampfes oder -nebels durch einen oberhalb des Flüssigkeitspegels im Inneren der Zerstäubungskammer angeordneten Turbulenzschild ausgebildet ist.

Zum Zwecke der Abtötung von gefährlichen Partikeln, wie Viren, Sporen, Bakterien etc. in Räumen bzw. zur Raum - und Flächendesinfektion wird der durch eine derartige bekannte Zerstäubungsvorrichtung abgegebene Flüssigkeitsdampf oder - nebel in einem Raum oder einer Umgebung einwirken gelassen. Es wird dabei z.B. eine zur Desinfektion geeignete Flüssigkeit in Form von Tröpfchen als Aerosol gleichmäßig in der Umgebungsluft verteilt, wodurch sie in der Luft und auf Flächen z.B. auf infektiöse Partikel trifft, welche durch die desinfizierende Wirkung der Flüssigkeit unschädlich gemacht werden.

Als nachteilig hat sich bisher die Tröpfchengröße und deren Größenverteilung erwiesen, die durch herkömmliche Zerstäubungsvorrichtungen nur sehr schlecht steuerbar sind. Beim Zerstäubungsvorgang durch Ultraschallwandler treten neben kleinen Tröpfchen auch größere auf, die einen relativ starken Niederschlag auf Oberflächen hervorrufen, der unerwünscht ist, weil er Feuchtigkeit entstehen lässt, die in vielerlei Hinsicht nachteilig sein kann. Empfindliche Oberflächen können dadurch zerstört werden oder es kann ein Korrosionsprozess an wertvollen Gegenständen oder Instrumenten in Gang gesetzt werden.

Je kleiner die Tröpfchengröße gehalten werden kann, umso weniger Feuchtigkeit bzw. Flüssigkeitsniederschlag entsteht in jener Umgebung, auf welche die Zerstäubungsvorrichtung einwirken soll.

Aus der GB 525 736 A geht eine Zerstäubungsvorrichtung mit einer Düse hervor, durch die Flüssigkeit aus einem Behälter unter Einwirkung eines Luftstromes in vertikaler Richtung nach oben zerstäubt wird, wo ein hohlkegelförmiges Rohrstück vorgesehen ist, durch dessen oberes Ende der Flüssigkeitsnebel austritt. Oberhalb der oberen Öffnung des Rohrstückes ist eine konusförmige Umlenkvorrichtung angeordnet, welche den aus dem Rohrstück austretenden Flüssigkeitsnebelstrom in Richtung nach unten lenkt, wonach dieser an der Außenseite der Umlenkvorrichtung vorbei nach oben ausströmen kann.

Ferner ist in der DE 10 2005 019 686 B3 eine Prozesskammer beschrieben, die mit einer Flüssigkeitswanne und die Flüssigkeit in Flüssigkeitsnebel umwandelnden Hochfrequenzschallvorrichtung versehen ist. Der Flüssigkeitsnebel wird zu einem Flüssigkeitsfallschacht umgelenkt, der zu einer Transportvorrichtung hin eine Verjüngung seines lichten Querschnittes aufweist. Aufgrund der keilförmigen Ausgestaltung des Fallschachtes wird durch den Rückstau der Flüssigkeitsnebel homogen und verdichtet. Im oberen Bereich der Prozesskammer ist ein schräg nach unten angeordnetes Prallelement ausgebildet, das Tropfen auffängt und zurückrieseln lässt.

Aus der WO 2009/013843 A1 geht eine tragbare Nebelerzeugungsvorrichtung hervor, in der oberhalb einer Flüssigkeitsoberfläche eines Behälters mit einem Ultraschallwandler ein Umlenkelement angeordnet ist, an dem aus der Flüssigkeit emporgeschleuderte Flüssigkeitsteilchen wieder in die Flüssigkeit zurückgeleitet werden. Der entstehende Flüssigkeitsnebel wird über ein Rohr abgeleitet.

Aufgabe der Erfindung ist es, eine Zerstäubungsvorrichtung der eingangs genannten Art anzugeben, mit deren Hilfe ein Flüssigkeitsnebel mit möglichst kleinem Tröpfchendurchmesser in die Umgebung abgegeben werden kann.

Erfindungsgemäß wird dies dadurch erreicht, dass der Turbulenzschild einen horizontalen Wandabschnitt und einen an diesen anschließenden, in Richtung zur Flüssigkeitsoberfläche geneigten Wandabschnitt aufweist.

An dieser Umlenkungsvorrichtung bildet sich ein Niederschlag aus Tröpfchen mit größerem Durchmesser aus, wodurch ein Flüssigkeitsnebel mit deutlich kleineren Tröpfchen verbleibt.

Auf diese Weise kann der durchschnittliche Durchmesser der Flüssigkeitströpfchen auf kleiner als 1µm verringert werden, sodass ein als sehr trocken empfundener Flüssigkeitsnebel entsteht, der daher in weiterer Folge als Trockendampf bezeichnet wird, der auf den zu behandelnden Flächen keinen Feuchtigkeitsfilm ausbildet, der zu Schimmel- oder Rostbildung führen könnte.

Im Unterschied zu herkömmlichen Geräten auf Dampfbasis muss aber die Flüssigkeit bei der Erzeugung des Trockendampfes nicht erhitzt werden.

Um ein sehr effizientes Beseitigen von größeren Tröpfchen zu erreichen, weist erfindungsgemäß der Turbulenzschild einen horizontalen Wandabschnitt und einen an diesen anschließenden, in Richtung zur Flüssigkeitsoberfläche geneigten Wandabschnitt auf, wobei der geneigte Wandabschnitt besonders dafür geneigt ist, den erzeugten Trockendampf in Richtung zur Austrittsöffnung umzulenken und gleichzeitig das Abtrennen der größeren Tröpfchen zu ermöglichen, wobei abgeschiedenen Tröpfchen z.B. in eine Wanne zurückfließen oder tropfen, in der die Flüssigkeit aufgenommen ist.

Insbesondere wenn gemäß einer weiteren Ausführungsform der Erfindung der geneigte Wandabschnitt sich oberhalb des zumindest einen Verneblers, der im Bodenbereich der Zerstäubungskammer angeordnet ist, erstreckt, ermöglicht dies eine besonders starke Herabsetzung des Anteils an großen Tröpfchendurchmessern im erzeugten Trockendampf.

Dabei hat sich als besondere bevorzugt herausgestellt, wenn der geneigte Wandabschnitt gegenüber der Horizontalen um den Winkel β1 geneigt ist, wobei β1 in einem Bereich von 10° bis 45° liegt.

Um den von den größeren Tröpfchen befreiten Flüssigkeitsnebel abzuleiten, wird eine Durchzugsströmung eingestellt, welche das entstandene Aerosol in Bewegung versetzt.

Zu diesem Zweck ist gemäß einem weiteren Ausführungsbeispiel der Erfindung eine Öffnung in der Zerstäubungskammer ausgebildet, durch die hindurch ein Luftstrom über die Flüssigkeitsoberfläche geführt werden kann.

In vorteilhafter Weise kann dabei vorgesehen sein, dass das freie Ende des in die Zerstäubungskammer ragenden Turbulenzschilds unter Ausbildung eines Öffnungsquerschnitts in einem Abstand zu einer Seitenwand der Zerstäubungskammer angeordnet ist, sodass die Zerstäubungskammer bis auf den Öffnungsquerschnitt in einen oberen und einen unteren Bereich unterteilt ist.

Weiters kann im oberen Bereich der Zerstäubungskammer eine Druckvorrichtung angeordnet sein, über welche innerhalb der Zerstäubungskammer der Druck erhöht wird, um einen Luftstrom entstehen zu lassen.

In weiterer Ausbildung der Erfindung kann die Druckvorrichtung durch zumindest ein Gebläse gebildet sein, über das Luft aus der Umgebung in die Zerstäubungskammer geblasen wird, wodurch ein Luftstrom durch den Öffnungsquerschnitt hindurch erzeugt wird, welcher den aus der Flüssigkeit aufsteigenden Flüssigkeitsdampf oder - nebel in Richtung zur Austrittsöffnung bewegt.

Gemäß einer weiteren Ausführungsform kann die Austrittsöffnung an einem Ende eines Auslasskastens mit einem rechteckförmigen Rohrquerschnitt gebildet sein, der sich nach oben erstreckt. Durch diesen Auslasskasten wird der von größeren Tröpfchendurchmessern befreite Flüssigkeitsnebel in die Umgebung abgeleitet. Anstelle des rechteckförmigen Rohrquerschnitts kann aber auch ein anderer, z.B. ein kreisförmiger vorgesehen sein.

Eine weitere strömungstechnische Maßnahme zur Herabsetzung des Anteils an Flüssigkeitströpfchen mit großem Durchmesser kann dadurch gebildet sein, dass im Bereich der Austrittsöffnung ein Beschleunigungsprisma ausgebildet ist, das über den Flüssigkeitspegel hinausragt.

In besonders bevorzugter Weise kann das Beschleunigungsprisma aus zwei Schenkeln in Form eines umgekehrten V-Querschnitts ausgebildet sein, wobei die Schenkel in einem Winkel δ1 gegenüber der Vertikalen geneigt sind.

Um eine weitere Entfernung von Tröpfchen mit größerem Durchmesser aus dem Flüssigkeitsnebel zu ermöglichen, kann vorgesehen sein, dass das Beschleunigungsprisma mit seinem der Austrittsöffnung zugewandten Schenkel eine Querschnittsverengung mit der Weite a1 der Zerstäubungskammer ausbildet.

Schließlich wird eine Erhöhung der Austrittsgeschwindigkeit des Flüssigkeitsnebels dadurch gewährleistet, dass gemäß einer Weiterbildung der Erfindung an zumindest einer Seite des Austrittkastens ein rechteckförmiges Strömungshohlprofil angeordnet ist, dessen unteres Ende mit dem oberen Bereich der Zerstäubungskammer verbunden ist, sodass durch das Strömungshohlprofil ein Teilstrom der Druckvorrichtung geleitet wird.

Nachstehend wird die Erfindung anhand der in den Zeichnungen dargestellten Ausführungsbeispiele eingehend erläutert. Es zeigt dabei
Fig.1 eine Schrägansicht einer Ausführungsform der erfindungsgemäßen Zerstäubungsvorrichtung;
Fig.2 einen Schnitt quer zur Längsachse durch die Zerstäubungsvorrichtung gemäß Fig.1;
Fig.3 einen Schnitt BB durch die Ansicht gemäß Fig.4;
Fig.4 eine Seitenansicht der Ausführungsform gemäß Fig.1;
Fig.5 eine Draufsicht auf eine weitere Ausführungsform der erfindungsgemäßen Zerstäubungsvorrichtung;
Fig.6 einen Schnitt AA durch die Ansicht gemäß Fig.5;
Fig.7 ein vergrößerte Ansicht des Details B aus Fig.6 und
Fig.8 eine Schrägansicht der Ausführungsform gemäß Fig.5.

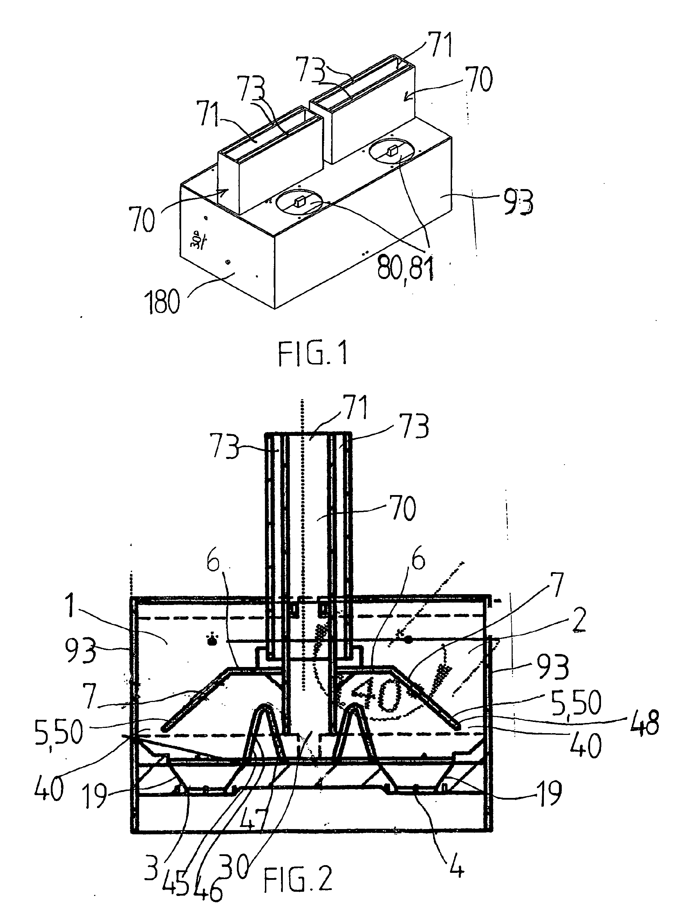

Fig.1 bis Fig.4 zeigen eine Ausführungsform einer erfindungsgemäßen Zerstäubungsvorrichtung, mit deren Hilfe eine Flüssigkeit zerstäubt und in Form von Flüssigkeitströpfchen an die Umgebung abgegeben werden kann, um z.B. einen Raum zu dekontaminieren oder desinfizieren, indem die Flüssigkeitströpfchen in der Luft schwebende oder an Oberflächen haftende Partikel so zerstören, dass sie für Menschen oder Tiere unwirksam werden, wodurch sie daran gehindert werden, Zellen zu befallen und dort ihre schädliche Wirkung zu entfalten. Es können aber auch z.B. Lebensmittel oder Computertastaturen von gefährlichen Keimen oder anderen Partikeln befreit werden, in dem diese dem von der erfindungsgemäßen Zerstäubungsvorrichtung abgegebenen Flüssigkeitsnebel oder -dampf ausgesetzt werden.

Ungeachtet der Art dieser Partikel, wie z.B. Viren, Pilze, Bakterien etc., stößt die Zerstäubungsvorrichtung einen stetigen Strom an Flüssigkeitströpfchen aus, die sich entsprechend verteilen und ihre desinfizierende Wirkung ausüben. Als zu zerstäubende Flüssigkeit kann z.B. Wasserstoffperoxid dienen, es können aber auch andere Flüssigkeiten oder Mischungen aus Flüssigkeiten oder auch reines Wasser zerstäubt werden, das keine desinfizierende Wirkung hat.

Die in Fig. 1 bis 4 gezeigte Zerstäubungsvorrichtung ist von einem Gehäuse 180 umgeben und weist in ihrem Inneren zwei Zerstäubungskammern 1, 2 zur Aufnahme der Flüssigkeit auf, welche Zerstäubungskammern 1, 2 spiegelsymmetrisch zur Mittelebene aufgebaut sind, wie aus Fig.2 ersichtlich ist. Es könnten aber genauso auch nur eine oder mehr als zwei Zerstäubungskammern vorgesehen sein, ohne dass dies einen Einfluss auf die Wirkungsweise der Zerstäubungsvorrichtung hätte.

Bei Betrieb sind die beiden Zerstäubungskammern 1, 2 bis zu einem vorbestimmbaren Flüssigkeitspegel mit Flüssigkeit gefüllt. Zu diesem Zweck hat der Bodenbereich der Zerstäubungskammern 1,2 eine wannenartige Form. Sobald der Pegel in einem gewissen Ausmaß unterschritten wird, ist ein Nachfüllvorgang erforderlich.

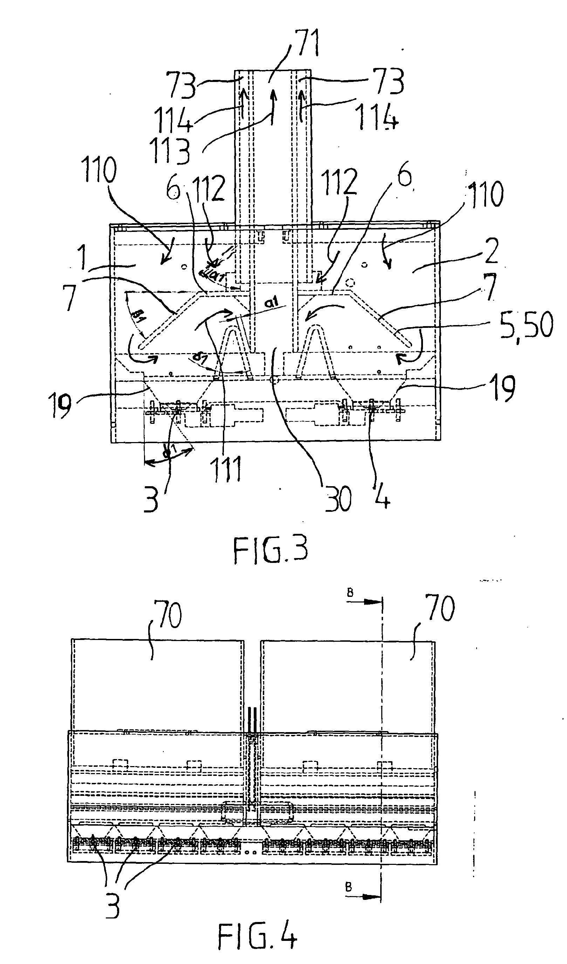

In den Zerstäubungskammern 1, 2 sind parallel zur Längsachse der Zerstäubungsvorrichtung jeweils eine Vielzahl von nebeneinander angeordneten Verneblern 3, 4 (Fig.4) vorgesehen, die z.B. im gezeigten Beispiel als Ultraschallvernebler ausgeführt sind. Es könnte im Rahmen der Erfindung aber auch eine andere Art der Zerstäubung, z.B. elektrostatischer Art angewandt werden.

Die Vernebler 3, 4, die z.B. als piezoelektrische Elemente ausgeführt sein können, sind dabei so in den Boden der Zerstäubungskammern 1, 2 eingelassen, dass sie mit ihren Schwingkörpern mit der Flüssigkeit in Berührung kommen und diese in Schwingung versetzen. Auf diese Weise findet ein Zerstäubungsvorgang statt, bei dem sich von der Flüssigkeitsoberfläche kleine Tröpfchen lösen, die in die Höhe geschleudert werden, wodurch ein Aerosol gebildet wird.

Zur besseren Wirkungsweise der Vernebler 3, 4 ist zwischen diesen und dem Kammerboden jeweils ein schallreflektierender Konus 19 ausgebildet, mit dem die von den Verneblern 3, 4 erzeugten Schallwellen eine Bündelung erfahren. Der Konus 19 kann aber auch weggelassen werden.

Als Konuswinkel Φ1 (Fig.3) hat sich ein Winkel im Bereich von 15° bis 80° als besonders bevorzugt herausgestellt, es können aber auch andere Winkelwerte angewandt werden.

Der entstehende Trockendampf gelangt über eine Austrittsöffnung 30, die an einem unteren Ende eines Auslasskastens 70, der im gezeigten Ausführungsbeispiel gemäß Fig.1 bis 4 in zwei Teilkästen unterteilt ist, mit einem rechteckförmigen Rohrquerschnitt ausgebildet ist, der sich nach oben erstreckt.

Erfindungsgemäß ist eine Vorrichtung zur Umlenkung des Flüssigkeitsdampfes oder -nebels 5 vorgesehen, die im Bereich oberhalb der Flüssigkeitsoberfläche angeordnet ist.

Im gezeigten Ausführungsbeispiel ist die Vorrichtung zur Umlenkung des Flüssigkeitsdampfes oder -nebels 5 durch einen oberhalb des Flüssigkeitspegels im Inneren der Zerstäubungskammern 1, 2 angeordneten Turbulenzschild 50 ausgebildet ist, der einen horizontalen Wandabschnitt 6 und einen an diesen anschließenden, in Richtung zur Flüssigkeitsoberfläche geneigten Wandabschnitt 7 aufweist. Es kann aber auch eine andere Art der Umlenkung vorgesehen sein.

Der geneigte Wandabschnitt 7 erstreckt sich oberhalb der Vernebler 3, 4, die im Bodenbereich der Zerstäubungskammer 1, 2 angeordnet sind, und ist gegenüber der Horizontalen um den Winkel β1 geneigt ist, wobei β1 in einem Bereich von 10° bis 45° liegt. In den Fig.2 und 3 ist der Winkel β1 mit 40° gewählt.

Von der Flüssigkeitsoberfläche aufsteigender Trockendampf wird durch den geneigten Wandabschnitt 7 in Richtung der Austrittsöffnung 30 umgelenkt. Dabei bleiben größere Tropfen an der Unterseite des geneigten Wandabschnitts 7 haften und rinnen entlang des geneigten Wandabschnitts 7 nach unten in Richtung der Flüssigkeit ab. Der restliche Nebel enthält auf diese Weise deutlich weniger Tröpfchen mit großem Durchmesser, sodass von einem Trockendampf gesprochen werden kann.

Das freie Ende des in die Zerstäubungskammern 1, 2 ragenden Turbulenzschilds 50 ist jeweils unter Ausbildung eines Öffnungsquerschnitts in einem Abstand zu einer Seitenwand 93 der Zerstäubungskammern 1, 2 angeordnet, sodass die Zerstäubungskammern 1, 2 bis auf den Öffnungsquerschnitt 40 in einen oberen und einen unteren Bereich unterteilt sind. Im oberen Bereich der Zerstäubungskammern ist jeweils eine Druckvorrichtung 80 angeordnet ist, über welche im Betrieb innerhalb der Zerstäubungskammern 1, 2 der Druck erhöht werden kann.

Die Druckvorrichtung 80 ist durch ein oder mehrere Gebläse 81 gebildet, über welche(s) Luft aus der Umgebung in die Zerstäubungskammer geblasen wird, wodurch ein Luftstrom durch den Öffnungsquerschnitt 40 hindurch erzeugt wird, welcher den aus der Flüssigkeit aufsteigenden Flüssigkeitsdampf oder -nebel in Richtung zur Austrittsöffnung 30 bewegt.

Im Bereich der Austrittsöffnung 30 ist ein Beschleunigungsprisma 45 ausgebildet, das über den Flüssigkeitspegel hinausragt und das aus zwei Schenkeln 46, 47 in Form eines umgekehrten V-Querschnitts ausgebildet ist, wobei die Schenkel 46, 47 in einem Winkel δ1 gegenüber der Mittelachse bzw. der Vertikalen geneigt sind. Der Winkel δ1 kann dabei in einem bevorzugten Bereich von 0° bis 60° liegen.

An diesem Beschleunigungsprisma 45 bilden zerstäubte Flüssigkeitströpfchen größeren Durchmessers ebenfalls einen Niederschlag aus, während die feineren Tröpfchen über dieses Beschleunigungsprisma 45 zur Austrittöffnung 30 gelangen. Zugleich bildet das Beschleunigungsprisma 45 mit seinem der Austrittsöffnung 30 zugewandten Schenkel 47 und dem unteren Ende des Auslasskastens 70 eine sich verjüngende Querschnittsverengung mit der Weite a1 am unteren Rand des Auslasskastens 70 aus. Die Austrittsöffnung 30 ist dabei ungefähr auf halber Höhe des Beschleunigungsprismas 45 angeordnet. Die Querschnittsverengungsweite a1 kann bevorzugt in einem Bereich von 1 mm bis 15mm gewählt sein, kann aber auch anders dimensioniert sein. Auch der Aufbau des Beschleunigungsprismas 45 kann im Rahmen der Erfindung anders ausgeführt sein, so kann dieses auch ohne Abstützung auf dem Beckenboden ausgebildet sein.

Insgesamt werden somit die zerstäubten Flüssigkeitströpfchen unter Einwirkung des von der Druckvorrichtung erzeugten Luftstromes (Pfeil 110) als ein Aerosolstrom (Pfeil 111) in Richtung zur Austrittsöffnung 30 bewegt und gelangen durch diese in den Austrittskasten 70, und durch diesen hindurch an das obere Ende des Austrittskastens 70 (Pfeil 113), wo eine Ausströmöffnung 71 ausgebildet ist, aus welcher der Trockendampf an die Umgebung abgegeben wird.

Das untere Ende des Austrittskastens 70 kann auch aufgeweitet sein, wie dies durch den Winkel α1 angedeutet ist, der im gezeigten Ausführungsbeispiel 0° ist. Als bevorzugt hat sich α1 in einem Bereich von 0° bis 45° herausgestellt.

Als zusätzliche Maßnahme, um den Austritt des Flüssigkeitsnebels aus der Ausströmöffnung 71 zu beschleunigen, sind an beiden Längsseiten des Austrittkastens 70 rechteckförmige Strömungshohlprofile 73 angeordnet, deren unteres Ende jeweils mit dem oberen Bereich der Zerstäubungskammern 1, 2 verbunden ist, sodass durch das Strömungshohlprofil 73 ein Teilstrom (Pfeil 112) der Druckvorrichtung 80 eingeleitet und durch dieses hindurchgeleitet wird (Pfeil 114), um an der Ausströmöffnung 71 des Austrittskastens 70 eine Sogwirkung entstehen zu lassen, welche die Ausströmgeschwindigkeit des Trockendampfes erhöht.

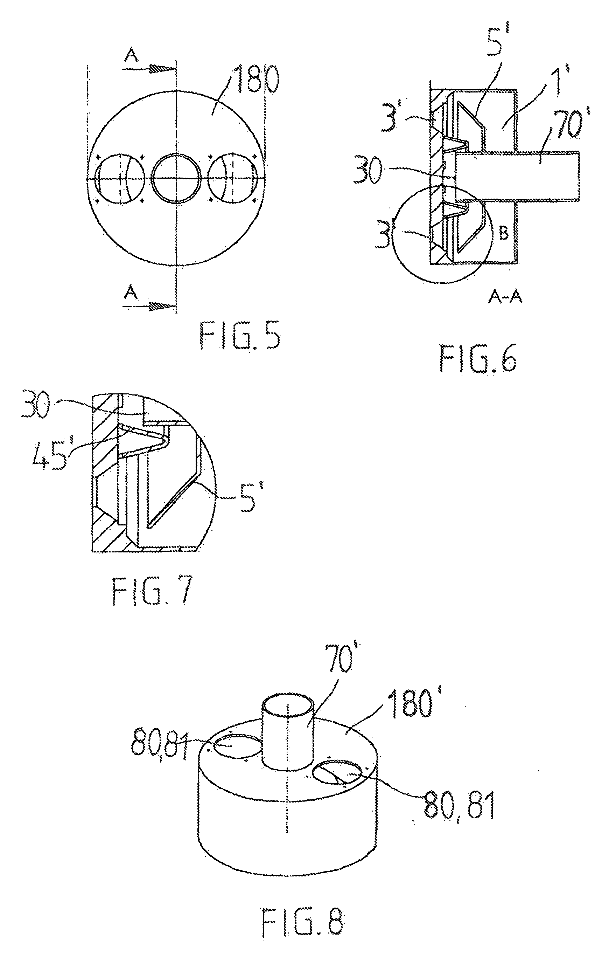

Fig.5 bis 8 zeigen ein weiteres Ausführungsbeispiel der erfindungsgemäßen Zerstäubungsvorrichtung mit einem zylindrischen Gehäuse 180', das eine ringförmige Zerstäubungskammer 1' aufnimmt. Eine hohlkegelstumpfförmige Umlenkungsvorrichtung 5' ist im Bereich oberhalb der Flüssigkeitsoberfläche angeordnet. Zusätzlich ist ein ringförmiges Beschleunigungsprisma 45' im Bereich der Austrittsöffnung 30 angeordnet, die durch das untere Ende eines hohlzylindrischen Austrittsrohrs 70' gebildet ist. Die Vernebler 3' liegen in einer kreisförmigen Anordnung vor. Die Funktionsweise entspricht jenem der in Fig.1 bis 4 dargestellten Ausführungsform. In gleicher Weise gelten auch die als bevorzugt angegebenen Winkelbereiche α1, β1, δ1, Φ1 und Weitenbereiche a1 für das Ausführungsbeispiel gemäß Fig. 5 bis 8 als bevorzugt, sodass die in Fig.5 bis 8 dargestellten Winkel lediglich als mögliche Variante anzusehen sind.

## Patentansprüche

1. Zerstäubungsvorrichtung mit einer Zerstäubungskammer (1,2;1') zur Aufnahme einer Flüssigkeit und zumindest einem Vernebler (3, 4; 3') zum Zerstäuben der Flüssigkeit zu Flüssigkeitströpfchen sowie einer Austrittsöffnung (30) für den Austritt des so erzeugten Flüssigkeitsdampfes oder -nebels aus der Zerstäubungskammer (1, 2; 1'), wobei der aus der Flüssigkeit aufsteigende Flüssigkeitsdampf oder - nebel über eine Druckvorrichtung (80) in Richtung zur Austrittsöffnung bewegt wird, und wobei zumindest eine Vorrichtung zur Umlenkung des Flüssigkeitsdampfes oder - nebels (5; 5') vorgesehen ist, welche im Bereich oberhalb der Flüssigkeitsoberfläche angeordnet ist, wobei im Bereich der Austrittsöffnung (30) ein Beschleunigungsprisma (45) ausgebildet ist, das über den Flüssigkeitspegel hinausragt und so angeordnet ist, dass eine Querschnittsverengung der Zerstäubungskammer (1, 2; 1') ausgebildet ist, und wobei die zumindest eine Vorrichtung zur Umlenkung des Flüssigkeitsdampfes oder -nebels durch einen oberhalb des Flüssigkeitspegels im Inneren der Zerstäubungskammer (1, 2) angeordneten Turbulenzschild (50) ausgebildet ist, **dadurch gekennzeichnet, dass** der Turbulenzschild (50) einen horizontalen Wandabschnitt (6) und einen an diesen anschließenden, in Richtung zur Flüssigkeitsoberfläche geneigten Wandabschnitt (7) aufweist.

2. Zerstäubungsvorrichtung nach Anspruch 1, **dadurch gekennzeichnet, dass** das Beschleunigungsprisma mit zwei Schenkeln (46, 47) in Form eines umgekehrten V-Querschnitts ausgebildet ist, wobei die Querschnittsverengung im Bereich des der Austrittsöffnung (30) zugewandten Schenkels des Beschleunigungsprismas (45) ausgebildet ist.

3. Zerstäubungsvorrichtung nach Anspruch 2, **dadurch gekennzeichnet, dass** die Schenkel (46, 47) in einem Winkel δ1 gegenüber der Vertikalen geneigt sind.

4. Zerstäubungsvorrichtung nach Anspruch 1, 2 oder 3, **dadurch gekennzeichnet, dass** die Querschnittsverengung der Zerstäubungskammer (1, 2) mit der Weite a1 ausgebildet ist.

5. Zerstäubungsvorrichtung nach einem der Ansprüche 1 bis 4, **dadurch gekennzeichnet, dass** der geneigte Wandabschnitt (7) sich oberhalb des zumindest einen Verneblers (3, 4), der im Bodenbereich der Zerstäubungskammer (1, 2) angeordnet ist, erstreckt.

6. Zerstäubungsvorrichtung nach Anspruch 5, **dadurch gekennzeichnet, dass** der geneigte Wandabschnitt (7) gegenüber der Horizontalen um den Winkel β1 geneigt ist, wobei β1 in einem Bereich von 10° bis 45° liegt.

7. Zerstäubungsvorrichtung nach Anspruch 5 oder 6, **dadurch gekennzeichnet, dass** das freie Ende des in die Zerstäubungskammer (1, 2) ragenden Turbulenzschilds (50) unter Ausbildung eines Öffnungsquerschnitts (48) in einem Abstand zu einer Seitenwand der Zerstäubungskammer (1, 2) angeordnet ist, sodass die Zerstäubungskammer (1, 2) bis auf den Öffnungsquerschnitt (48) in einen oberen und einen unteren Bereich unterteilt ist.

8. Zerstäubungsvorrichtung nach Anspruch 7, **dadurch gekennzeichnet, dass** die Druckvorrichtung (80) im oberen Bereich der Zerstäubungskammer angeordnet ist, über welche innerhalb der Zerstäubungskammer (1, 2) der Druck erhöht wird.

9. Zerstäubungsvorrichtung nach Anspruch 8, **dadurch gekennzeichnet, dass** die Druckvorrichtung durch zumindest ein Gebläse (81) gebildet ist, über das Luft aus der Umgebung in die Zerstäubungskammer (1,2) geblasen wird, wodurch ein Luftstrom durch den Öffnungsquerschnitt (48) hindurch erzeugt wird, welcher den aus der Flüssigkeit aufsteigenden Flüssigkeitsdampf oder -nebel in Richtung zur Austrittsöffnung (30) bewegt.

10. Zerstäubungsvorrichtung nach einem der vorhergehenden Ansprüche, **dadurch gekennzeichnet, dass** die Austrittsöffnung (30) an einem Ende eines Auslasskastens (70) mit einem rechteckförmigen Rohrquerschnitt gebildet ist, der sich nach oben erstreckt.

11. Zerstäubungsvorrichtung nach Anspruch 10, **dadurch gekennzeichnet, dass** an zumindest einer Seite des Auslasskastens (70) ein rechteckförmiges Strömungshohlprofil (73) angeordnet ist, dessen unteres Ende mit dem oberen Bereich der Zerstäubungskammer (1, 2) verbunden ist, sodass durch das Strömungshohlprofil (73) ein Teilstrom der Druckvorrichtung (80) geleitet wird.

## Claims

1. An atomization device with an atomization chamber (1, 2; 1') for receiving a liquid and at least one nebulizer (3, 4; 3') for atomizing the liquid into drops of liquid as well as an exit port (30) for discharging the vapor or mist of liquid thus generated from the atomization chamber (1, 2; 1'), wherein the vapor or mist of liquid ascending from the liquid is moved towards the exit port via a pressure device (80) and wherein at least one device for deflecting the vapor or mist of liquid (5; 5') is provided, which is arranged in the area above the liquid surface, an acceleration prism (45) being formed in the area of the exit port (30) that projects above the liquid level and is arranged so that a cross-sectional narrowing of the atomization chamber (1, 2; 1') is formed and said at least one device for deflecting the vapor or mist of liquid being formed by a turbulence screen (50) arranged above the liquid level in the interior of the atomization chambers (1, 2), **characterized in that** the turbulence screen (50) has a horizontal wall section (6) and a wall section (7) connected thereto and inclined towards the liquid surface.

2. The atomization device according to claim 1 **characterized in that** the acceleration prism is formed by two legs (46, 47) in the form of an inverted V cross section, the cross-sectional narrowing being formed in the area of the leg of the acceleration prism (45) facing the exit port (30).

3. The atomization device according to claim 2 **characterized in that** the legs (46, 47) are inclined with regard to the vertical at an angle of δ1.

4. The atomization device according to claim 1, 2 or 3 **characterized in that** the cross-sectional narrowing of the atomization chamber (1, 2) is formed having a width of a1.

5. The atomization device according to claim 1 to 4 **characterized in that** the inclined wall section (7) extends above the at least one nebulizer (3, 4) arranged in the bottom area of the atomization chamber (1, 2).

6. The atomization device according to claim 5 **characterized in that** the inclined wall section (7) is inclined at an angle of β1 with regard to the horizontal, with β1 being in the range of 10° to 45°.

7. The atomization device according to claim 5 or 6 **characterized in that** the free end of the turbulence screen (50) projecting into the atomization chamber (1, 2) is arranged at a distance from a side wall of the atomization chamber (1, 2) forming an opening cross-section (48), so that the atomization chamber (1, 2) is separated into an upper and a lower area except for the opening cross-section.

8. The atomization device according to claim 7 **characterized in that** a pressure device (80) is arranged in the upper area of the atomization chamber (1, 2), via which the pressure within the atomization chamber (1, 2) is increased.

9. The atomization device according to claim 8 **characterized in that** the pressure device is formed by at least one fan (81) via which air is blown into the atomization chamber (1, 2) from the surroundings, so that an airflow is created through the opening cross-section (48), which moves the vapor or mist of liquid ascending from the liquid towards the exit port (30).

10. The atomization device according to any one of the preceding claims **characterized in that** the exit port (30) is formed at one end of a discharge box (70) with a rectangular pipe cross-section extending upwards.

11. The atomization device according to claim 10 **characterized in that** a rectangular flow hollow profile (73) is arranged on at least one side of the discharge box (70), the lower end of which is connected to the upper part of the atomization chamber (1, 2), so that a partial flow of the pressure device (80) is led through the flow hollow profile (73).

## Revendications

1. Dispositif d'atomisation avec une chambre d'atomisation (1, 2; 1') pour recevoir un liquide et au moins un nébuliseur (3, 4 ; 3') pour atomiser le liquide pour obtenir des gouttes de liquide ainsi qu'un orifice de sortie (30) pour décharger de la chambre d'atomisation (1, 2 ; 1') le vapeur ou le brouillard de liquide généré ainsi, ledit vapeur ou brouillard de liquide qui monte du liquide étant transporté vers l'orifice de sortie par une dispositif de pression (80) et au moins un dispositif pour dévier le vapeur ou le brouillard de liquide (5 ; 5') étant fourni, ledit dispositif étant positionné dans un zone au-dessus de la surface du liquide, un prisme d'accélération (45), qui dépasse le niveau du liquide et est arrangé ainsi qu'une contraction de la section transversale de la chambre d'atomisation (1, 2; 1') est formée, étant constitué dans le zone de l'orifice de sortie (30), ledit au moins un dispositif pour dévier le vapeur ou le brouillard de liquide étant formé par un écran de turbulence (50) arrangé au-dessus du niveau du liquide à l'intérieur de la chambre d'atomisation (1, 2), **caractérisé en ce que** l'écran de turbulence (50) a une partie de paroi horizontale (6) et une partie de paroi (7) reliée à la partie horizontale (6) et penchée vers la surface du liquide.

2. Dispositif d'atomisation selon la revendication 1, **caractérisé en ce que** le prisme d'accélération est formé par deux branches (46, 47) en forme d'une section transversale en forme d'un V renversé, la contraction de la section transversale étant formée dans le zone de la branche du prisme d'accélération (45) orientée vers l'orifice de sortie (30).

3. Dispositif d'atomisation selon la revendication 2, **caractérisé en ce que** les branches (46, 47) sont inclinées à un angle δ1 par rapport à la verticale.

4. Dispositif d'atomisation selon la revendication 1, 2 ou 3, **caractérisé en ce que** la contraction de la section transversale de la chambre d'atomisation (1, 2) est formée avec une largeur a1.

5. Dispositif d'atomisation selon les revendications 1 à 4, **caractérisé en ce que** la section de paroi penchée (7) s'étend au-dessus ledit au moins un nébuliseur (3, 4) situé dans le zone inférieur de la chambre d'atomisation (1, 2).

6. Dispositif d'atomisation selon la revendication 5, **caractérisé en ce que la** section de paroi penchée (7) est inclinée par rapport à la horizontale à un angle β1, β1 variant entre 10° et 45°.

7. Dispositif d'atomisation selon la revendication 5 ou 6, **caractérisé en ce que** l'extrémité libre de l'écran de turbulence (50) qui fait saillie dans la chambre d'atomisation (1, 2) est arrangée à une distance d'une paroi latérale de la chambre d'atomisation (1, 2), formant une section transversale d'un orifice (48) ainsi que la chambre d'atomisation (1, 2) est divisée en un zone supérieur et un zone inférieur, à l'exception de la section transversale d'orifice.

8. Dispositif d'atomisation selon la revendication 7, **caractérisé en ce qu'**un dispositif de pression (80) est arrangé dans le zone supérieur de la chambre d'atomisation (1, 2), augmentant la pression dans la chambre d'atomisation (1, 2).

9. Dispositif d'atomisation selon la revendication 7, **caractérisé en ce que** le dispositif de pression est formé par au moins un ventilateur (81) qui insuffle de l'air de l'environnement dans la chambre d'atomisation (1, 2), ainsi qu'un courant d'air à travers la section transversale d'orifice (48) est créé, transportant le vapeur ou le brouillard de liquide qui monte de la surface de liquide vers l'orifice de sortie (30).

10. Dispositif d'atomisation selon une des revendications précédentes, **caractérisé en ce que** l'orifice de sortie (30) est formé à une extrémité d'une boîte de sortie (70) avec une section transversale rectangulaire de conduit qui s'étend vers le haut.

11. Dispositif d'atomisation selon la revendication 7, **caractérisé en ce qu**'un profile creux rectangulaire (73) d'écoulement est arrange à au moins un côté de ladite boîte de sortie (70), l'extrémité inférieure dudit profile étant reliée à la partie supérieure de la chambre d'atomisation (1, 2) ainsi qu'un courant partiel provenant du dispositif de pression (80) est dirigé à travers le profile creux (73) d'écoulement.
